# EUROPEAN PATENT APPLICATION

(11) **EP 4 663 627 A1**
(43) Date of publication of application: **17.12.2025**
(21) Application number: 24753248.4
(22) Date of filing: 02.02.2024
(51) Int. Cl.: C07D 207/452, C07D 207/456

(54) **AMINE HYDROCHLORIDE HAVING MALEIMIDE GROUP, AND METHOD FOR PRODUCING SAME**

(30) Priority: 06.02.2023 JP 2023016157
(71) Applicant: NOF Corporation, Shibuya-ku Tokyo 150-6019 (JP)
(72) Inventor: KAWADA, Aiko, Kawasaki-shi, Kanagawa 210-0865 (JP); KINOSHITA, Shuhei, Kawasaki-shi, Kanagawa 210-0865 (JP); KIMURA, Takato, Kawasaki-shi, Kanagawa 210-0865 (JP); ISOBE, Yuuki, Tokyo 150-6012 (JP)
(74) Representative: Hoffmann Eitle
(86) International application number: PCT/JP2024/003427
(87) International publication number: WO 2024/166805

(57) **Abstract**

Provided are: an amine hydrochloride having a maleimide group and having less hydrochloride adducts; and a method for producing the amine hydrochloride. The present invention involves: deprotecting, by trifluoroacetic acid, a protected amine having a maleimide group and represented by formula (2) to obtain an amine trifluoroacetate having a maleimide group and represented by formula (3) (step (a)); and performing salt exchange by mixing the amine trifluoroacetate with an organic solvent solution of hydrogen chloride to obtain an amine hydrochloride having a maleimide group and represented by formula (1) (step (b)).

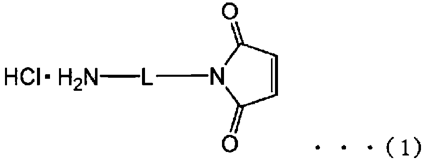

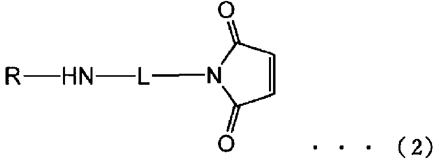

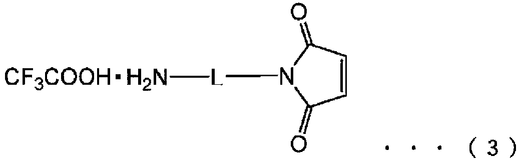

## Description

### TECHNICAL FIELD

The present invention relates to an amine hydrochloride having a maleimide group and a method for producing the same.

### BACKGROUND ART

Conventionally, it has been known that a maleimide group has high reactivity, and in particular, easily reacts with thiol or conjugated diene. Therefore, maleimide compounds are useful as coupling agents with these compounds, and are widely used as raw materials for resins, agricultural chemicals, and pharmaceutical products.

For example, a compound having a maleimide group and an amine is useful as a candidate for a coupling agent when the maleimide group is introduced into an electron withdrawing group such as a carboxylic acid or a sulfonic acid. In this regard, since the amine easily reacts with the maleimide group, an intramolecular reaction or an intermolecular reaction proceeds. Therefore, for example, a technique described in Non Patent Literature 1 is known as a technique for stabilizing a compound having a maleimide group and an amine by converting the amine into an amine salt using an acid.

In Non Patent Literature 1, a maleimide group is used at a peptide introduction point in synthesis of a cholesterol-polyethylene glycol-peptide. As a method for introducing a maleimide group, cholesterol-polyethylene glycol reacts with an amine trifluoroacetate having a maleimide group.

The amine trifluoroacetate having a maleimide group is obtained by reacting (tert-butoxycarbonyl)-ethylenediamine with 3-maleimide propionic acid in the presence of EDC (1-ethyl-3-(3-dimethylaminopropyl) carbodiimide) hydrochloride, and then deprotecting a t-butoxycarbonyl group with trifluoroacetic acid. After converting cholesterol-polyethylene glycol into carboxylic acid chloride, by reacting the carboxylic acid chloride with the amine trifluoroacetate having a maleimide group in the presence of a base, the maleimide group is introduced into cholesterol-polyethylene glycol.

As described above, the amine trifluoroacetate having a maleimide group can be used for a reaction with a compound having a strong electron withdrawing property, such as carboxylic acid chloride, at a terminal of a target compound. However, in a case of reacting with a target compound having a carboxyl group at a terminal, which is inferior in the electron withdrawing property to trifluoroacetic acid, the trifluoroacetic acid also reacts with an amine to cause reaction inhibition to form trifluoroacetamide.

Examples of a method for preventing the reaction inhibition include a method described in Patent Literature 1. In Patent Literature 1, a t-butoxy carbonyl group protector of an amino having a maleimide group is deprotected using a 1,4-dioxane solution of hydrogen chloride to obtain an amine hydrochloride having a maleimide group.

### PRIOR ART DOCUMENTS

### PATENT LITERATURE

Patent Literature 1: US8,034,558B

### NON-PATENT LITERATURE

Non Patent Literature 1: ACS Omega 2020, 5, 5508 page to 5519 page

### SUMMARY OF INVENTION

### OBJECT TO BE ACHIEVED BY INVENTION

However, when an amine hydrochloride having a maleimide group is synthesized by the method of Patent Literature 1, a compound in which hydrogen chloride is added to the maleimide group (hereinafter, abbreviated as a "hydrogen chloride adduct") is by-produced, and a purity of the amine hydrochloride having a maleimide group is reduced.

Therefore, a high-purity amine hydrochloride having a maleimide group with a few hydrogen chloride adducts and a method for producing the same are desired.

The present invention has been made in view of the above object, and an object thereof is to provide an amine hydrochloride having a maleimide group with a few hydrogen chloride adducts, and a method for producing the same.

### MEANS FOR ACHIEVING THE OBJECT

Thus, the present invention is as follows.
[1] A method for producing an amine hydrochloride having a maleimide group represented by a formula (1), the method including:
   a step (a) of deprotecting an amine protector having a maleimide group represented by a formula (2) with trifluoroacetic acid to obtain an amine-trifluoroacetate having a maleimide group represented by a formula (3); and
   a step (b) of mixing the amine-trifluoroacetate obtained in the step (a) with an organic solvent solution of hydrogen chloride to perform salt exchange to obtain the amine hydrochloride having the maleimide group represented by the formula (1). 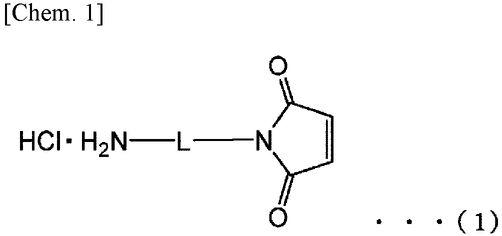
   (In the formula (1), L represents a linker connecting the maleimide group and an amino group.) 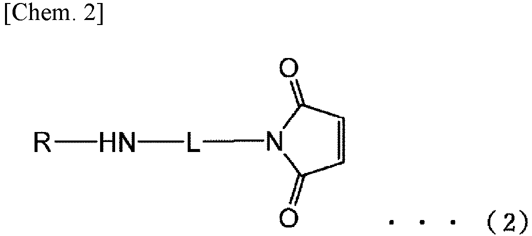
   (In the formula (2), L represents a linker connecting the maleimide group and a protected amino group, and R represents a protecting group for the amino group.) 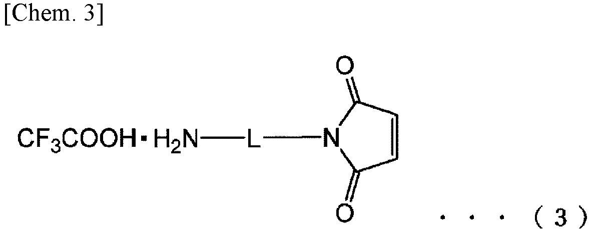
   (In the formula (3), L represents a linker connecting the maleimide group and an amino group.)
[2] The method for producing an amine hydrochloride having a maleimide group according to [1], in which
   the protecting group R has a structure of a formula (4), (5), (6), (7), (8), or (9). 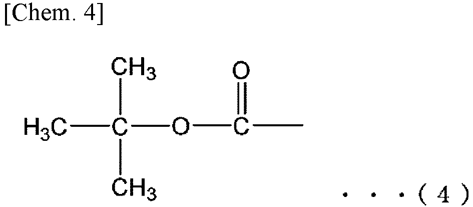 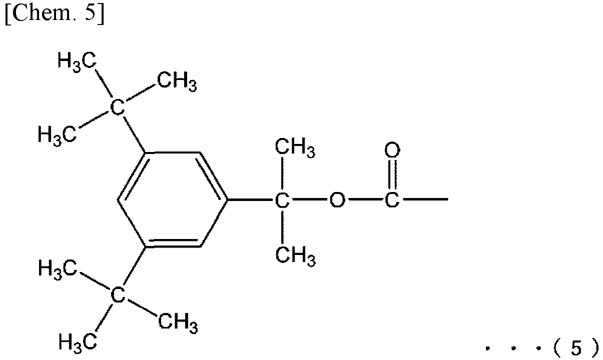 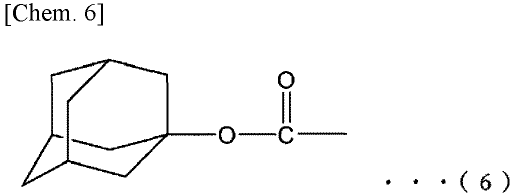 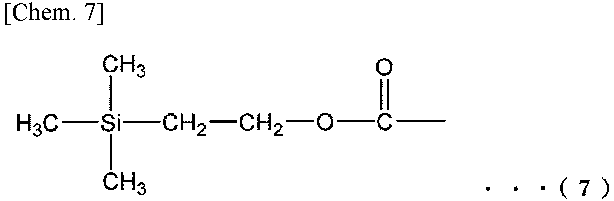 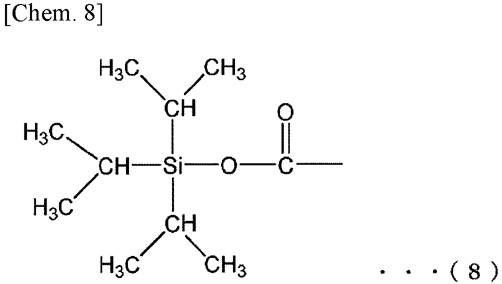 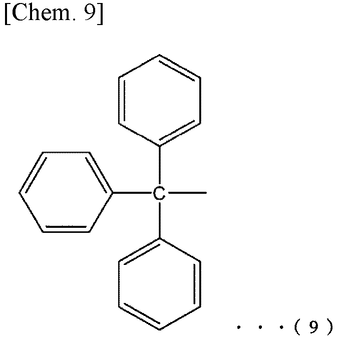
[3] The method for producing an amine hydrochloride having a maleimide group according to [2], in which
   the linker L has a structure of a formula (10).
   [Chem. 10]

   -X₁-Y-X₂- ... (10)

   (In the formula (10), X₁ and X₂ are each independently a hydrocarbon group, and Y represents an amide bond.)
[4] The method for producing an amine hydrochloride having a maleimide group according to [3], in which
   X₁ and X₂ are a divalent hydrocarbon group having 2 to 10 carbon atoms.
[5] The method for producing an amine hydrochloride having a maleimide group according to any one of [1] to [4], in which
   an equivalent of the organic solvent solution of hydrogen chloride used in the step (b) is 1.5 equivalents or more and 12 equivalents or less with respect to the amino group of the amine-trifluoroacetate.
[6] An amine hydrochloride having a maleimide group represented by a formula (1), including:
   a hydrogen chloride adduct of an amine hydrochloride represented by a formula (11), in which
   a content of the hydrogen chloride adduct is 0.001% by mass or more and 2.5% by mass or less. 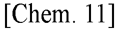 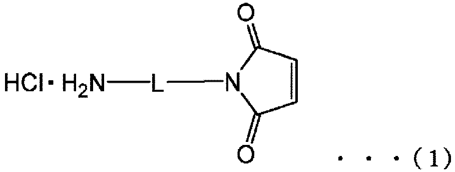 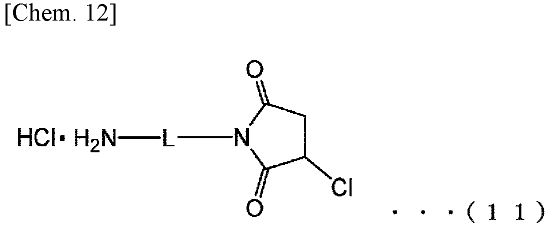 (In the formula (1) and the formula (11), L represents a linker connecting the maleimide group and an amino group.)

### EFFECTS OF INVENTION

Since the step (b) of performing the salt exchange using the organic solvent solution of hydrogen chloride in the production method of the present invention can be easily executed, a high-purity amine hydrochloride having a maleimide group can be obtained without performing a complicated process such as ion exchange chromatography or column chromatography. That is, according to the present invention, a high-purity amine hydrochloride having a maleimide group, which is difficult to obtain by the technique in the conventional art, can be easily obtained by two steps including the deprotection and the salt exchange.

### EMBODIMENTS FOR CARRYING OUT THE INVENTION

The present invention is an amine hydrochloride having a maleimide group represented by a formula (1), including a hydrogen chloride adduct of an amine hydrochloride represented by a formula (11), in which a content of the hydrogen chloride adduct is 0.001% by mass or more and 2.5% by mass or less.

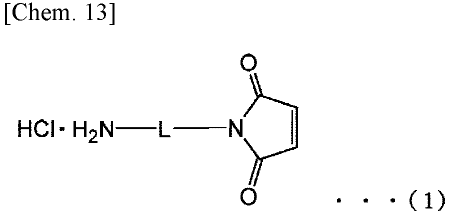

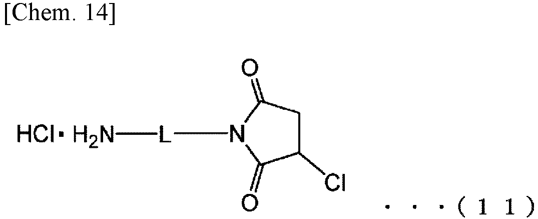

Here, in the formula (1) and the formula (11), L represents a linker connecting the maleimide group and an amino group. A structure of the linker L may be a straight-chain structure, a branched structure, or a cyclic structure.

In a preferred embodiment, the linker L may contain a hydrocarbon group or may have an amide bond. The hydrocarbon group may not have an unsaturated bond or may have an unsaturated bond, and the number of the unsaturated bonds is preferably two or less, and more preferably one or less. The linker L is preferably a substituent having a straight-chain structure, a branched structure, or a cyclic structure containing a hydrocarbon group and an amide bond. The number of carbon atoms in the hydrocarbon group is preferably 2 to 20, and more preferably 4 to 16.

In a particularly preferred embodiment, the linker L has a structure of a formula (10).
[Chem. 15]

-X₁-Y-X₂- ... (1 0)

Here, in the formula (10), X₁ and X₂ are each independently a divalent hydrocarbon group, and Y represents an amide bond.

Each of the hydrocarbon groups constituting X₁ and X₂ may have a straight-chain structure, a branched structure, or a cyclic structure. Each of the hydrocarbon groups may not have an unsaturated bond or may have an unsaturated bond, and the number of the unsaturated bonds is preferably two or less, and more preferably one or less. The number of carbon atoms in each of the hydrocarbon groups is preferably 2 to 10.

The hydrogen chloride adduct of the formula (11) is an impurity generated in a step of performing salt exchange on the amine hydrochloride having a maleimide group represented by the formula (1). A content of the hydrogen chloride adduct in the amine hydrochloride having a maleimide group of the present invention is 0.001% by mass or more and 2.5% by mass or less. The content of the hydrogen chloride adduct in the amine hydrochloride having a maleimide group is preferably 1.0% by mass or less, and more preferably 0.5% by mass or less. In addition, since the hydrogen chloride adduct is by-produced as long as a hydrogen chloride source is present during the salt exchange, the content is 0.001% by mass or more from a viewpoint of actual production, but is always 0.01% by mass or more.

### (Amine Protector Having Maleimide Group Represented by Formula (2))

An amine protector having a maleimide group used as a raw material in the present invention has a structure of a formula (2).

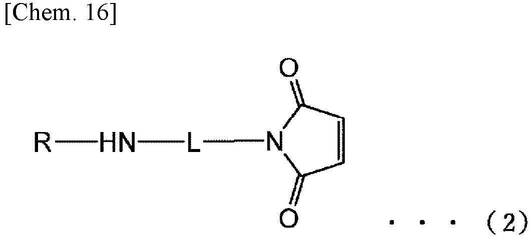

Here, L represents a linker connecting the maleimide group and a protected amino group, and R represents a protecting group for the amino group.

R shown in the formula (2) is a protecting group of an amino group that can be deprotected with trifluoroacetic acid, and is preferably a protecting group of a formula (4) to a formula (9). Among these, in the formula (4), since only volatile isobutene and carbon dioxide are by-produced during the deprotection, a post-treatment is also simple, and thus the protecting group of the foormula (4) is most preferable.

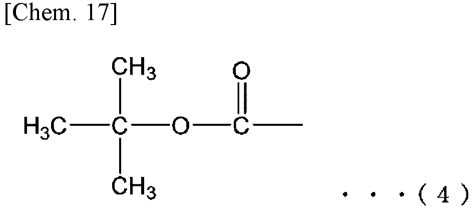

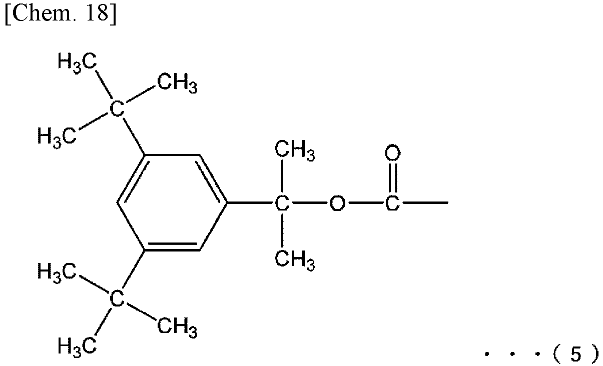

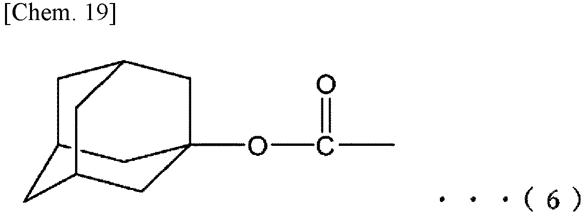

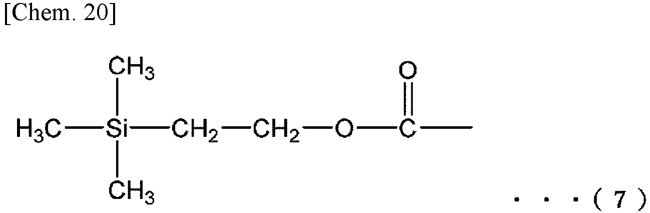

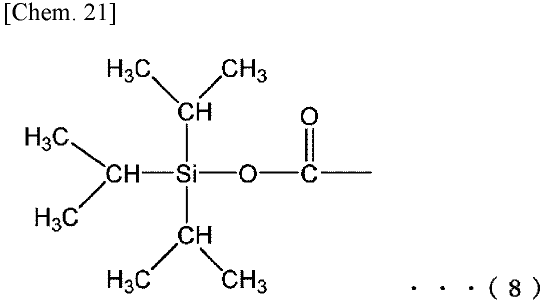

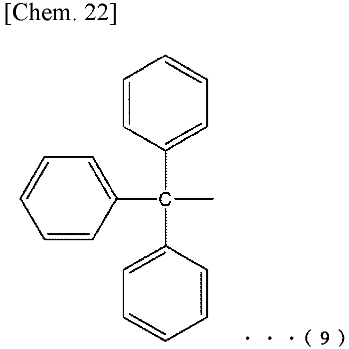

As the amine protector having a maleimide group represented by the formula (2), for example, an amine protector having a structure represented by a formula (12), a formula (13), or a formula (14) is particularly preferable. The amine protector represented by the formula (12) can be produced by reacting N-(tert-butoxycarbonyl)-1,2-diaminoethane with N-succinimidyl maleimidopropionate. The amine protector represented by the formula (13) can be produced by reacting N-(tert-butoxycarbonyl)-5-methyl-1,5-diaminoethane with N-succinimidyl maleimidobutanate. The amine protector represented by the formula (14) can be produced by reacting N-(tert-butoxycarbonyl)-1,2-diaminoethane with N-succinimidyl-4-(N-maleimidomethyl) cyclohexanecarboxylate.

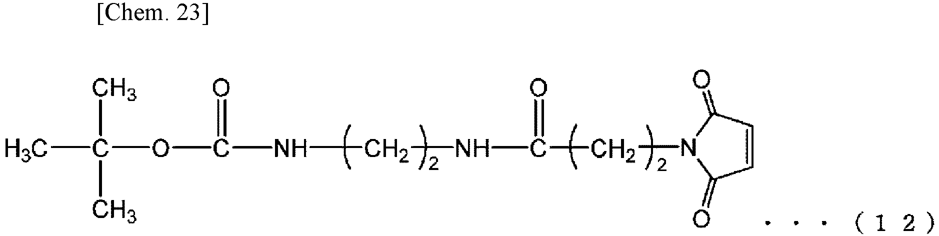

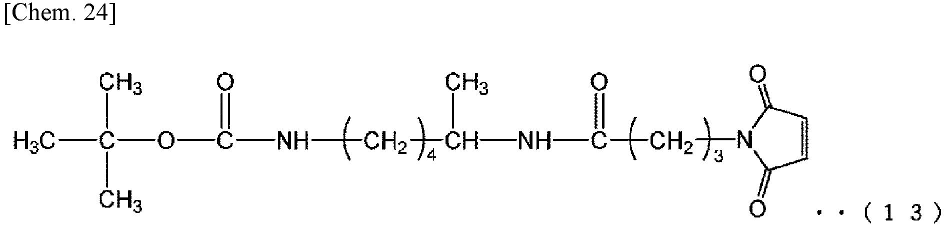

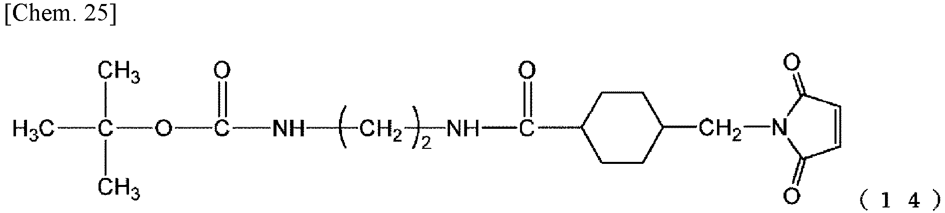

### [Method for Producing Amine Hydrochloride Having Maleimide Group Represented by Formula (1)]

### (Step (a))

In a step (a), the amine protector having a maleimide group represented by the formula (2) is deprotected with trifluoroacetic acid to obtain an amine-trifluoroacetate having a maleimide group represented by the formula (3).

As a deprotection method, a system using trifluoroacetic acid which is an organic acid or a system using hydrochloric acid which is an inorganic acid is known. Here, since hydrogen chloride contained in the hydrochloric acid has high nucleophilicity of a chloride ion and easily undergoes an addition reaction with a maleimide group, the content of the hydrogen chloride adduct represented by the formula (11) increases. Therefore, hydrogen chloride is not preferable as an acid to be used for deprotection of an amine protector having a maleimide group.

Therefore, in the present invention, trifluoroacetic acid is used for the deprotection of the amine protector having a maleimide group. This is because trifluoroacetic acid anion has lower nucleophilicity than that of the chloride ion, so that the addition reaction with the maleimide group is difficult to occur, and as a result, a purity of a target product increases.

After the step (a) of performing the deprotection, since there is a concern that the amine may be added to the maleimide group, it is necessary to form an amine salt under acidic conditions. That is, after the step (a) of performing the deprotection, it is not preferable to perform purification by a step of removing a counter ion of the amine, for example, by column chromatography.

In the deprotection reaction in the step (a), a ratio of the trifluoroacetic acid to the amine protector having a maleimide group is preferably 2 times by weight to 5 times by weight, and more preferably 2.2 times by weight to 3 times by weight.

A reaction temperature of the deprotection reaction in the step (a) is preferably 15°C to 40°C, and more preferably 20°C to 35°C.

A solvent for the deprotection reaction in the step (a) is preferably a halogen-based solvent such as dichloromethane or chloroform, or an ether-based solvent such as cyclopentyl methyl ether.

In the deprotection in the step (a), the amine trifluoroacetate having a maleimide group may be crystallized.

### (Step (b))

In a step (b), the amine-trifluoroacetate obtained in the step (a) is mixed with an organic solvent solution of hydrogen chloride to perform the salt exchange, thereby obtaining the amine hydrochloride having a maleimide group represented by the formula (1). The organic solvent solution of hydrogen chloride used in the step (b) uses carboxylic acid ester, alcohol, or alkyl ether in which hydrogen chloride gas is soluble and which is liquid under temperature conditions of step (b). Examples of the carboxylic acid ester, alcohol, and alkyl ether include the following organic solvents.

### ·Carboxylic Acid Ester:

The number of carbon atoms of an alkyl group of a carboxylic acid-derived structure is preferably 1 to 2. The number of carbon atoms of an alkyl group of an alcohol-derived structure is preferably 1 to 4, and more preferably 2 to 3.

### ·Alcohol:

Alcohol is primary or secondary alcohol, and the number of carbon atoms of an alkyl group is preferably 1 to 3, and more preferably 2 to 3.

### ·Alkyl Ether:

An ether of alkyl groups, an ether of an alkyl group and a cycloalkyl group, or a cyclic ether is preferred. The number of carbon atoms of the alkyl group is preferably 1 to 4. The number of carbon atoms of the cycloalkyl group is preferably 5 to 6. The cyclic ether preferably has 1 to 2 ether bonds and has 4 to 5 carbon atoms.

The organic solvent of the organic solvent solution of hydrogen chloride used in the step (b) is preferably ethyl acetate, ethanol, isopropanol, 1,4-dioxane, or cyclopentyl methyl ether, and most preferably ethyl acetate or 1,4-dioxane.

When an aqueous hydrochloric acid solution is used as a hydrogen chloride source in the salt exchange in the step (b), it is difficult to isolate the amine hydrochloride having a maleimide group as a crystal from the aqueous solution because of high water solubility. Even if the isolation is possible, since the number of steps is large and the time of exposure to the hydrogen chloride source is long, an amount of by-produced impurities such as a hydrogen chloride adduct increases.

An adding amount of the organic solvent solution of hydrogen chloride in the salt exchange in the step (b) is preferably reduced as much as possible because hydrogen chloride is added to the maleimide group. On the other hand, when the adding amount of the organic solvent solution of hydrogen chloride is too small, the salt exchange from trifluoroacetic acid to hydrogen chloride is difficult to sufficiently proceed. From such a viewpoint, the adding amount of the organic solvent solution of hydrogen chloride is preferably 1.5 equivalents or more and 12 equivalents or less, more preferably 1.5 equivalents or more and 8.2 equivalents or less, and particularly preferably 2.0 equivalents or more and 4.0 equivalents or less with respect to an amino group of the target compound (amine-trifluoroacetate having a maleimide group). A mass mol concentration of hydrogen chloride in the reaction solution is preferably 0.011 mol/kg or more and 3.2 mol/kg or less, more preferably 0.011 mol/kg or more and 2.19 mol/kg or less, and particularly preferably 0.14 mol/kg or more and 1.1 mol/kg or less.

A temperature of the salt exchange in the step (b) is preferably -100°C or higher and 40°C or lower, more preferably -10°C or higher and 20°C or lower, and most preferably 0°C or higher and 10°C or lower from a viewpoint of reducing an amount of the by-produced hydrogen chloride adduct.

After the step (b), the amine hydrochloride having a maleimide group is recovered from the solution by a step including any of concentration, crystallization, drying, and the like. Since the amine hydrochloride having a maleimide group is always low in solubility in an organic solvent, when a crystal is precipitated during the salt exchange, the crystal can be recovered by filtration or the like. In this way, a high-purity amine hydrochloride having a maleimide group can be obtained without performing a complicated process such as ion exchange chromatography or column chromatography.

### EXAMPLES

### [Step of Producing Amine Protector Having Maleimide Group (Compound (A))]

N-(tert-butoxycarbonyl)-1,2-diaminoethane (manufactured by Tokyo Chemical Industry Co., Ltd.) and N-succinimidyl-3-maleimidopropionate (manufactured by Tokyo Chemical Industry Co., Ltd.) were reacted in acetonitrile for 2 hours. A crystal obtained by subjecting the obtained reaction solution to desolventization was washed with a solvent or purified by column chromatography to obtain an amine protector having a maleimide group (compound (A)).

### <Example 1>

### (Step (a))

Trifluoroacetic acid (manufactured by Kanto Chemical Co., Inc., 2.0 g) was added to a dichloromethane (manufactured by Kanto Chemical Co., Inc., 5.0 g) solution of the amine protector having a maleimide group (compound (A), 1.0 g, purity: 100% by mass), and the mixture was stirred under nitrogen at 25°C for 3 hours. After stirring, it was confirmed by thin layer chromatography (hereinafter, referred to as "TLC") that a residual amount of the amine protector having a maleimide group was 5% by mass or less. The obtained reaction solution was added dropwise to dichloromethane (300 g) cooled to 5°C for crystallization, and a crystal of an amine trifluoroacetate (compound (B)) was recovered by filtration.

### (Step (b))

Ethyl acetate (manufactured by Kanto Chemical Co., Inc., 451 mg) was added to the obtained crystal (50 mg) of the compound (B), and an ethyl acetate solution of 4M hydrogen chloride (manufactured by FUJIFILM Wako Pure Chemical Corporation, 341 mg) was added at 25°C, and the mixture was stirred for 30 minutes. Here, an equivalent of the ethyl acetate solution of hydrogen chloride was set to 8.2 equivalents with respect to an amino group of the amine protector having a maleimide group. The precipitated crystal was separated by filtration and washed with ethyl acetate (manufactured by Kanto Chemical Co., Inc.) to obtain a white crystal of an amine hydrochloride having a maleimide group (compound (C)).

A purity of the amine hydrochloride having a maleimide group (compound (C)) obtained as described above was 98.5% by mass, and a content of a hydrogen chloride adduct (compound (D)) was 1.3% by mass.

### [Method for Analyzing Amine Hydrochloride Having Maleimide Group and Hydrogen Chloride Adduct]

The purity of the amine hydrochloride having a maleimide group and the content of the hydrogen chloride adduct are evaluated by high performance liquid chromatography with charged aerosol detection (HPLC-CAD). The content of the hydrogen chloride adduct can also be confirmed by nuclear magnetic resonance spectroscopy (NMR).

### <Example 2>

### (Step (a))

Trifluoroacetic acid (200 mg) was added to an amine protector having a maleimide group (compound (A), 100 mg, purity: 88.7% by mass) in dichloromethane (200 mg), and the mixture was stirred under nitrogen at 25°C for 3 hours. An amine-trifluoroacetate (compound (B)) thus obtained was confirmed by TLC that a residual amount of the amine protector having a maleimide group was 5% by mass or less.

### (Step (b))

Ethyl acetate (75.0 g) was added to the reaction solution obtained in the step (a) and cooled to 10°C, a 1,4-dioxane solution of 4M hydrogen chloride (manufactured by Tokyo Chemical Industry Co., Ltd., 229 mg) was added, and the mixture was stirred for 30 minutes. Here, an equivalent of the 1,4-dioxane solution of hydrogen chloride was set to 3.0 equivalents with respect to an amino group of the amine protector having a maleimide group. The precipitated crystal was separated by filtration and washed with ethyl acetate (4.8 g) to obtain a white crystal of an amine hydrochloride having a maleimide group (compound (C)).

A purity of the amine hydrochloride having a maleimide group (compound (C)) obtained as described above was 94.8% by mass, and a content of a hydrogen chloride adduct (compound (D)) was 2.5% by mass.

### <Example 3>

Trifluoroacetic acid (4.0 g) was added to an amine protector having a maleimide group (compound (A), 2.0 g, purity: 100% by mass) in dichloromethane (10 g), and the mixture was stirred under nitrogen at 25°C for 3 hours. An amine-trifluoroacetate (compound (B)) thus obtained was confirmed by TLC that a residual amount of the amine protector having a maleimide group was 5% by mass or less. The obtained reaction solution was added dropwise to dichloromethane (600 g) cooled to 5°C to crystallize the amine-trifluoroacetate (compound (B)), and the compound (B) was recovered by filtration.

### (Step (b))

Ethyl acetate (59 g) was added to the obtained compound (B) (1.3 g) and cooled to 10°C, and an ethyl acetate solution (8.2 g) of 4M hydrogen chloride was added, and the mixture was stirred for 30 minutes. Here, an equivalent of the ethyl acetate solution of hydrogen chloride was set to 8.2 equivalents with respect to an amino group of the amine protector having a maleimide group. The precipitated crystal was recovered by filtration and then washed with ethyl acetate (58 g) to obtain a white crystal of an amine hydrochloride having a maleimide group (compound (C)).

A purity of the amine hydrochloride having a maleimide group (compound (C)) obtained as described above was 99.8%, and a content of a hydrogen chloride adduct (compound (D)) was 0.2% by mass.

### <Example 4>

### (Step (a))

Trifluoroacetic acid (1.2 g) was added to an amine protector having a maleimide group (compound (A), 600 mg, purity: 99.9% by mass) in dichloromethane (1.2 g), and the mixture was stirred under nitrogen at 25°C for 3 hours. An amine-trifluoroacetate (compound (B)) thus obtained was confirmed by TLC that a residual amount of the amine protector having a maleimide group was 5% by mass or less.

### (Step (b))

Ethyl acetate (9.0 g) was added to the reaction solution obtained in the step (a) and cooled to 10°C, an ethyl acetate solution of 4M hydrogen chloride (371 mg) was added, and the mixture was stirred for 30 minutes. Here, an equivalent of the ethyl acetate solution of hydrogen chloride was set to 2.5 equivalents with respect to an amino group of the amine protector having a maleimide group (compound (A)). The precipitated crystal was recovered by filtration and then washed three times with ethyl acetate (2.2 g) to obtain a white crystal of an amine hydrochloride having a maleimide group(compound (C)).

A purity of the amine hydrochloride having a maleimide group (compound (C)) obtained as described above was 97.4% by mass, and a content of a hydrogen chloride adduct (compound (D)) was 0.001% by mass.

Structures of the compounds (A), (B), (C), and (D) are as follows.

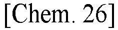

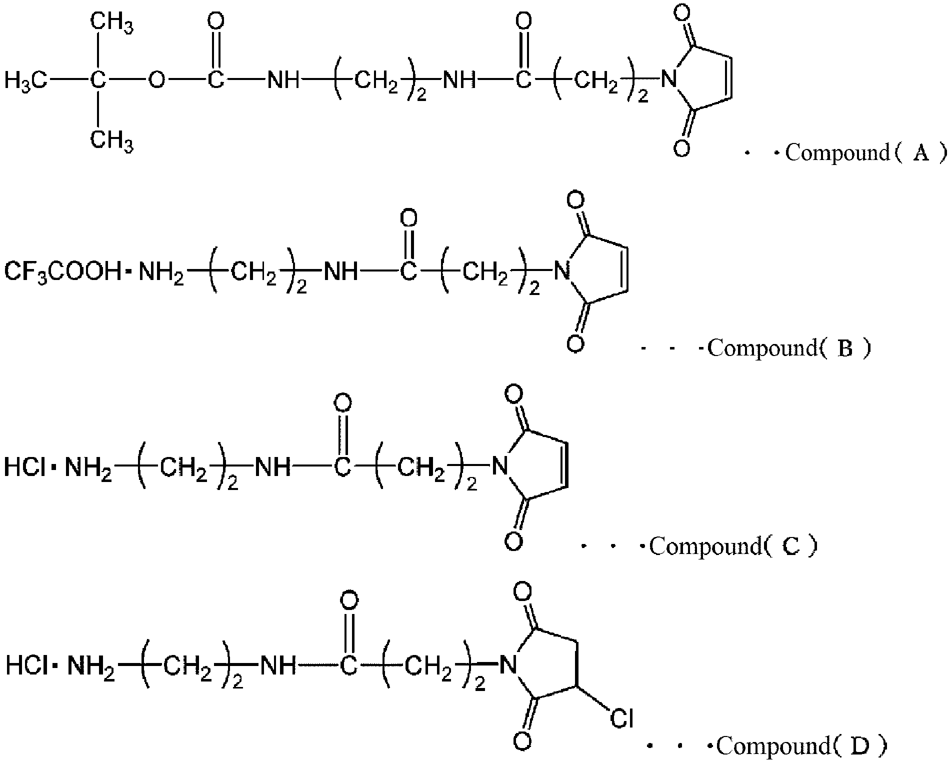

### <Comparative Example 1>

A 6N aqueous hydrochloric acid solution (manufactured by Kanto Chemical Co., Inc., 585 mg, 5.9 times by weigh (10 equivalents)/compound (A)) was added to an amine protector having a maleimide group (compound (A), 100 mg, purity: 88.7% by mass) in dichloromethane (200 mg), and the mixture was stirred under nitrogen at 25°C for 3 hours. It was confirmed by TLC that a residual amount of the amine protector having a maleimide group (compound (A)) was 5% by mass or less. After water was distilled off, ethyl acetate (4.8 g) was added, and precipitated viscous solid was separated by filtration to obtain an amine hydrochloride having a maleimide group (compound (C)).

A purity of the amine hydrochloride having a maleimide group (compound (C)) obtained as described above was 91.9% by mass, and a content of a hydrogen chloride adduct (compound (D)) was 3.1% by mass.

### <Comparative Example 2>

A 1,4-dioxane solution of 4M hydrogen chloride (manufactured by Tokyo Chemical Industry Co., Ltd., 313 mg) was added to an amine protector having a maleimide group (compound (A), 50 mg, purity: 100% by mass) in isopropanol (manufactured by Kanto Chemical Co., Inc., 1.0 g), and the mixture was stirred under nitrogen at 25°C for 3 hours. Here, an equivalent of the 1,4-dioxane solution of hydrogen chloride was set to 8.2 equivalents with respect to an amino group of the amine protector having a maleimide group (compound (A)). When a residual amount of the amine protector having a maleimide group was confirmed by TLC, 40% by mass remained.

### <Comparative Example 3>

A 1,4-dioxane solution of 4M hydrogen chloride (manufactured by Tokyo Chemical Industry Co., Ltd., 7.7 g) was added to an amine protector having a maleimide group (compound (A), 500 mg, purity: 88.7% by mass) in isopropanol (5.0 g), and the mixture was stirred under nitrogen at 25°C for 3 hours. An equivalent of the 1,4-dioxane solution of hydrogen chloride was set to 20 equivalents with respect to an amino group of the amine protector having a maleimide group (compound (A)). It was confirmed by TLC that a residual amount of the amine protector having a maleimide group was 5% by mass or less. The mixture was diluted with isopropanol (25.0 g), hexane (25.5 g) was added, and a precipitated crystal was separated by filtration to obtain an amine hydrochloride having a maleimide group (compound (C)).

A purity of the amine hydrochloride having a maleimide group (compound (C)) obtained as described above was 83.3% by mass, and a content of a hydrogen chloride adduct (compound (D)) was 16.7% by mass.

### <Comparative Example 4>

Trifluoroacetic acid (200 mg) was added to an amine protector having a maleimide group (compound (A), 100 mg, purity: 88.7% by mass) in dichloromethane (200 mg), and the mixture was stirred under nitrogen at 25°C for 3 hours. It was confirmed by TLC that a residual amount of the amine protector having a maleimide group was 5% by mass or less.

Dichloromethane (1.5 g) was added, and a 6N aqueous hydrochloric acid solution (535 mg, 10 equivalents/compound (A)) was further added, the mixture was stirred for 30 minutes. This solution was concentrated, ethyl acetate was added, and precipitated viscous solid was separated by filtration to obtain an amine hydrochloride having a maleimide group (compound (C)).

A purity of the amine hydrochloride having a maleimide group (compound (C)) obtained as described above was 91.7% by mass, and a content of a hydrogen chloride adduct (compound (D)) was 3.0% by mass.

Results of Examples 1 to 4 and Comparative Example 1 to 4 are shown in Tables 1 and 2.

**[Table 1]**

| Name of step | Item | Example 1 | Example 2 | Example 3 | Example 4 |
|---|---|---|---|---|---|
| Step (a) Deprotection | Type of deprotecting agent | Trifluoroacetic acid | Trifluoroacetic acid | Trifluoroacetic acid | Trifluoroacetic acid |
| | Adding amount (times by weight) | 2.0 (5.5 equivalents) | 2.0 (5.5 equivalents) | 2.0 (5.5 equivalents) | 2.0 (5.5 equivalents) |
| Step (b) Salt exchange | Type of salt exchange reagent | Ethyl acetate solution of hydrogen chloride | 1,4-Dioxane solution of hydrogen chloride | Ethyl acetate solution of hydrogen chloride | Ethyl acetate solution of hydrogen chloride |
| | Adding amount (equivalent) | 8.2 | 3.0 | 8.2 | 2.5 |
| | Temperature (°C) | 25 | 10 | 10 | 10 |
| Product | Purity (% by mass) of amine hydrochloride having maleimide group | 98.5 | 94.8 | 99.8 | 97.4 |
| | Content (% by mass) of chlorine adduct | 1.3 | 2.5 | 0.2 | 0.001 |

**[Table 2]**

| Step | Item | Comparative Example 1 | Comparative Example 2 | Comparative Example 3 | Comparative Example 4 |
|---|---|---|---|---|---|
| Deprotection | Type of deprotecting agent | Hydrochloric acid | 1,4-Dioxane solution of hydrogen chloride | 1,4-Dioxane solution of hydrogen chloride | Trifluoroacetic acid |
| | Adding amount (times by weight) | 5.9 (10 equivalents) | 6.3 (8.2 equivalents) | 15.4 (20 equivalents) | 2.0 (5.5 equivalents) |
| Salt exchange | Type of salt exchange reagent | - | - | - | Hydrochloric acid |
| | Adding amount (equivalent) | - | - | - | 10 |
| | Temperature (°C) | 25 | 25 | 25 | 25 |
| Product | Purity (% by mass) of amine hydrochloride having maleimide group | 91.9 | * | 83.3 | 91.7 |
| | Content (% by mass) of chlorine adduct | 3.1 | * | 16.7 | 3.0 |

| | | | | | |
|---|---|---|---|---|---|
| *Measurement was not performed because deprotection did not proceed sufficiently and 40% by mass of the amine protector having a maleimide group (compound (A)) remained. | | | | | |

In Examples 1 and 2, as shown in Table 1, after deprotection with trifluoroacetic acid, the salt exchange was performed using the organic solvent solution of hydrogen chloride. As a result, an amount of the hydrogen chloride adduct was reduced to 2.5% by mass or less.

In Example 3, as compared with Example 1, a reaction temperature of the salt exchange was lowered, but the amount of the by-produced hydrogen chloride adduct could be further significantly reduced.

In Example 4, as compared with Example 1, the reaction temperature of the salt exchange was lowered, and the equivalent of the organic solvent solution of hydrochloric acid was also lowered, but the amount of the by-produced hydrogen chloride adduct could be further significantly reduced.

On the other hand, in Comparative Examples 1 and 3, as compared with Examples 1 to 4, the amount of the by-produced hydrogen chloride adduct increased, and the purity of the amine hydrochloride having a maleimide group decreased. In Comparative Example 2, the progress of deprotection was insufficient. Accordingly, it was found that the use of trifluoroacetic acid as a deprotecting agent is preferable to the use of hydrochloric acid or the organic solvent solution of hydrogen chloride.

Further, also in Comparative Example 4, as compared with Examples 1 to 4, the amount of the by-produced hydrogen chloride adduct was increased, and the purity of the amine hydrochloride having a maleimide group was lowered. In addition, the obtained solid was viscous. Therefore, it was found that in order to perform the salt exchange while reducing the amount of the by-produced hydrogen chloride adduct, the use of the organic solvent solution of hydrogen chloride is preferable to the use of hydrochloric acid.

### INDUSTRIAL APPLICABILITY

The production method of the present invention can be used as a method for producing an amine hydrochloride having a maleimide group for which a high-purity product is required. The amine hydrochloride having a maleimide group produced by the production method of the present invention is useful as a raw material for resins, agricultural chemicals, drugs, and the like.

Although the present invention has been described in detail and with reference to specific embodiments, it will be apparent to those skilled in the art that various changes and modifications can be made without departing from the spirit and scope of the present invention.

The present application is based on a Japanese patent application (Japanese Patent Application No. 2023-016157) filed on February 6, 2023, the contents of which are incorporated herein by reference.

## Claims

1. A method for producing an amine hydrochloride having a maleimide group represented by a formula (1), the method comprising:
a step (a) of deprotecting an amine protector having a maleimide group represented by a formula (2) with trifluoroacetic acid to obtain an amine-trifluoroacetate having a maleimide group represented by a formula (3); and
a step (b) of mixing the amine-trifluoroacetate obtained in the step (a) with an organic solvent solution of hydrogen chloride to perform salt exchange to obtain the amine hydrochloride having the maleimide group represented by the formula (1). 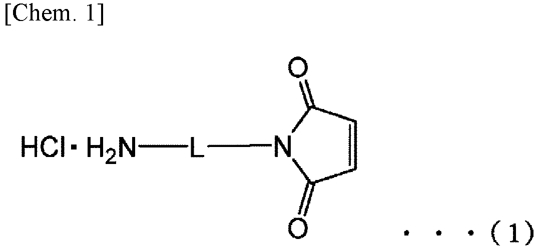
(In the formula (1), L represents a linker connecting the maleimide group and an amino group.) 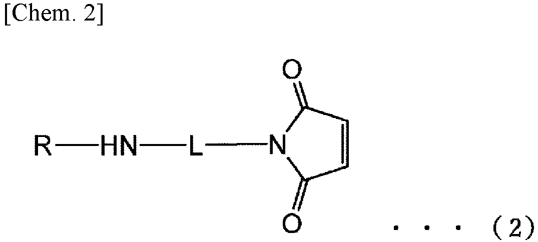
(In the formula (2), L represents a linker connecting the maleimide group and a protected amino group, and R represents a protecting group for the amino group.) 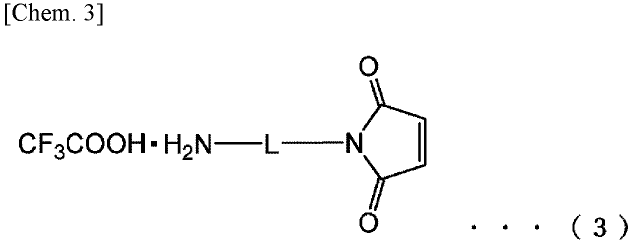
(In the formula (3), L represents a linker connecting the maleimide group and an amino group.)

2. The method for producing an amine hydrochloride having a maleimide group according to claim 1, wherein
the protecting group R in the formula (2) has a structure of a formula (4), (5), (6), (7), (8) or (9).

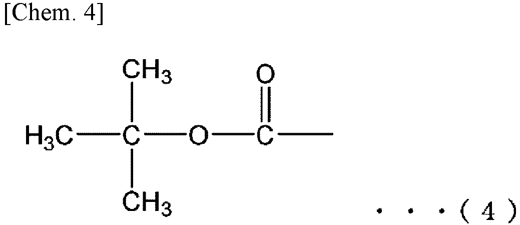

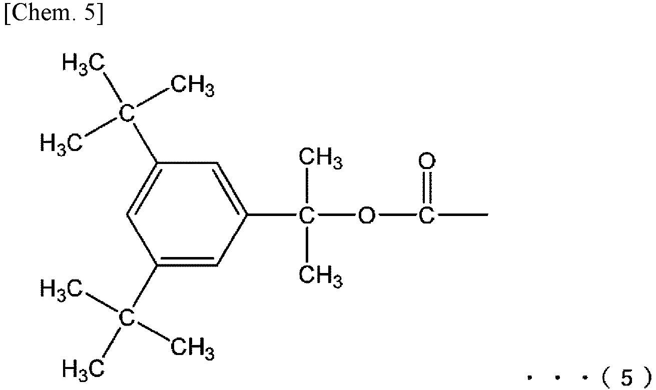

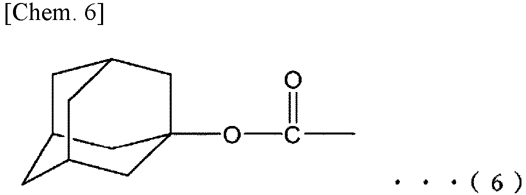

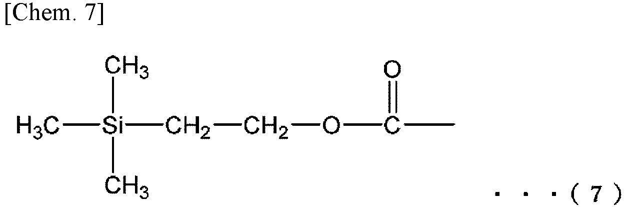

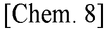

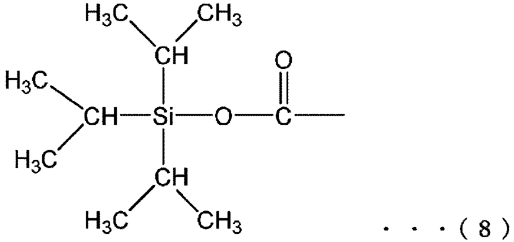

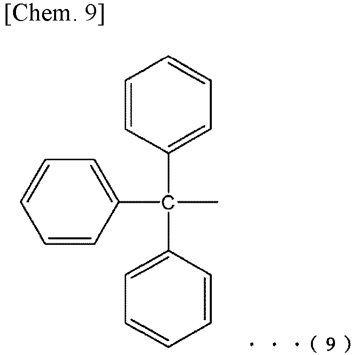

3. The method for producing an amine hydrochloride having a maleimide group according to claim 2, wherein
the linker L has a structure of a formula (10).
[Chem. 10]
-X₁-Y-X₂- ... (10)
(In the formula (10), X₁ and X₂ are each independently a divalent hydrocarbon group, and Y represents an amide bond.)

4. The method for producing an amine hydrochloride having a maleimide group according to claim 3, wherein
X₁ and X₂ are a hydrocarbon group having 2 to 10 carbon atoms.

5. The method for producing an amine hydrochloride having a maleimide group according to any one of claims 1 to 4, wherein
an equivalent of the organic solvent solution of hydrogen chloride used in the step (b) is 1.5 equivalents or more and 12 equivalents or less with respect to the amino group of the amine-trifluoroacetate.

6. An amine hydrochloride having a maleimide group represented by a formula (1), comprising:
a hydrogen chloride adduct of an amine hydrochloride represented by a formula (11), wherein
a content of the hydrogen chloride adduct is 0.001% by mass or more and 2.5% by mass or less. 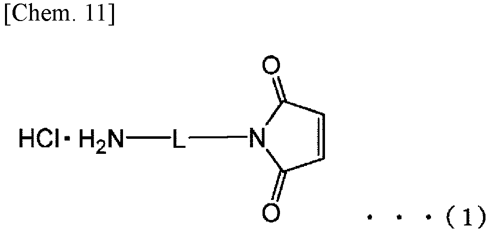 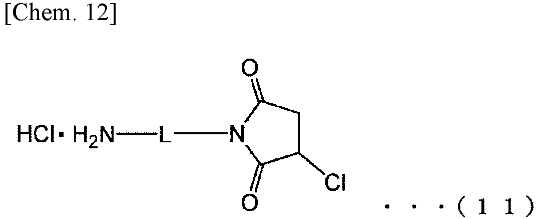
(In the formula (1) and the formula (11), L represents a linker connecting the maleimide group and an amino group.)
